# FASCICULE DE BREVET EUROPEEN

(11) **EP 2 382 182 B1**
(45) Date de publication et mention de la délivrance du brevet: **14.11.2012**
(21) Numéro de dépôt: 10707592.1
(22) Date de dépôt: 27.01.2010
(51) Int. Cl.: C07C 213/06, C07C 213/10

(54) **PROCEDE DE PURIFICATION DE LA FRACTION AZEOTROPIQUE GENEREE LORS DE LA SYNTHESE DE L'ACRYLATE DE N,N-DIMETHYL AMINOETHYLE**
VERFAHREN ZUR REINIGUNG DER WÄHREND DER SYNTHESE VON N,N-DIMETHYLAMINOETHYLACRYLAT ERZEUGTEN AZEOTROPEN FRAKTION
METHOD FOR PURIFYING THE AZEOTROPIC FRACTION GENERATED DURING THE SYNTHESIS OF N,N-DIMETHYLAMINOETHYL ACRYLATE

(30) Priorité: 27.01.2009 FR 0950471
(43) Date de publication de la demande: 02.11.2011
(73) Titulaire: Arkema France, 92700 Colombes (FR)
(72) Inventeur: PAUL, Jean-Michel, F-57070 Metz (FR); GRAIRE, Coralie, F-57890 Porcelette (FR); RIEHL, Audrey, F-69007 Lyon (FR)
(74) Mandataire: Bonnel, Claudine
(86) Numéro de dépôt international: PCT/FR2010/050119
(87) Numéro de publication internationale: WO 2010/086547

(56) Documents cités:
- EP-A- 0 906 902
- FR-A- 2 811 986

## Description

La présente invention concerne la fabrication de l'acrylate de N,N-diméthyl aminoéthyle par réaction de transestérification d'un acrylate d'alkyle par le N,N-diméthyl aminoéthanol, et a plus particulièrement pour objet un procédé de purification de la fraction azéotropique générée lors de cette réaction, permettant son recyclage sur l'unité de production de l'acrylate d'alkyle.

L'acrylate de N,N-diméthyl aminoéthyle (désigné ci-après par ADAME) répondant à la formule (I) :

H₂C=CH-COOCH₂CH₂N(CH₃)₂ (I)

est obtenu par réaction de transestérification entre un acrylate d'alkyle léger de formule (II) : CH₂=CH-CODR₁ dans laquelle R₁ représente le radical méthyle ou éthyle, et le N,N-diméthyl aminoéthanol (DMAE), selon le schéma réactionnel suivant :

CH2=CH-COOR₁ + HO-CH₂CH₂N(CH₃)₂ ⇔ ADAME + R₁OH (1)

La réaction s'effectue en général en présence d'un excès d'acrylate d'alkyle léger et la réaction est déplacée dans le sens de la formation de l'ADAME en distillant l'alcool léger R₁OH sous forme d'un azéotrope acrylate léger / alcool R₁OH, qui peut être avantageusement recyclé, si sa qualité le permet vers l'atelier de production de l'acrylate léger, ledit acrylate léger étant fabriqué par estérification directe de l'acide acrylique avec l'alcool R₁OH.

Selon le document EP 906 902, le mélange azéotropique résultant de la distillation, est purifié sur une résine échangeuse d'ions réduisant ainsi sa teneur en azote liée à la présence de sous-produits aminés susceptibles de se former lors de la réaction de transestérification.

Lors de la synthèse de l'ADAME par réaction de transestérification, il se forme généralement de l'acétaldéhyde CH₃CHO comme sous-produit. L'acétaléléhyde peut alors réagir avec l'alcool R₁OH pour conduire à un acétal, du dialcoxyéthane, selon la réaction :

CH₃CHO + 2 R₁OH ⇔ CH₃CH(OR₁)₂ + H₂O (2)

Le dialcoxyéthane est le diéthoxyéthane ou le diéthoxyéthane selon que l'ADAME est préparé à partir d'acrylate de méthyle, ou d'acrylate d'éthyle.

L'acétaldéhyde et le dialcoxyétane produits lors de la synthèse de l'ADAME se retrouvent majoritairement dans la fraction azéotropique acrylate léger / alcool R₁OH, et contribuent, de ce fait, à la polluer.

Lors du recyclage de la fraction azéotropique à l'atelier de production de l'acrylate de méthyle ou de l'acrylate d'éthyle, l'acétaldéhyde, au contact du méthanol ou de l'éthanol et du catalyseur acide présents pour effectuer la réaction d'estérification, se transforme en dialcoxyéthane qui s'ajoute au dialcoxyéthane déjà présent dans la fraction azéotropique recyclée. Il en résulte la production d'acrylate de méthyle ou d'acrylate d'éthyle fortement pollué par du dialcoxyéthane. C'est particulièrement le cas pour l'acrylate d'éthyle ; le diéthoxyéthane ayant une température d'ébullition du même ordre que celle de l'acrylate d'éthyle.

Il y a donc nécessité de trouver un procédé permettant de supprimer tout ou partie de l'acétaldéhyde et du dialcoxyéthane présents dans la fraction azéotropique générée lors de la synthèse de l'acrylate de N,N-diméthyl aminoéthyle à partir d'un acrylate léger, ladite fraction étant susceptible d'être recyclée à l'atelier de production de l'acrylate léger, faute de quoi l'acrylate léger se trouvera fortement pollué par du dialcoxyéthane. La purification de l'acrylate léger s'avère alors difficile, l'écart entre la température d'ébullition de l'acrylate léger et celle du dialcoxyéthane étant relativement faible.

De ce fait, il est difficile de produire un acrylate d'alkyle exempt de dialcoxyéthane, si on recycle à l'atelier de production de l'acrylate d'alkyle des fractions acrylate/alcool qui en contiennent ou qui renferment de l'acétaldéhyde qui en est le précurseur.

L'élimination de l'acétaldéhyde dans les fractions acrylate léger/alcool par simple réduction par utilisation d'un agent réducteur du type par exemple NaBH₄, LiAlH₄, sulfite de sodium, n'est pas envisageable dans le cas présent. En effet, en présence d'acrylate léger, l'utilisation de ces agents réducteurs entraînerait la formation en grandes quantités d'adduits de Michaël (méthoxy propionate de méthyle ou éthoxypropionate d'éthyle). Ceci se traduirait par une perte en acrylate d'alkyle et en alcool léger (méthanol ou éthanol).

Un des objectifs de la présente invention est donc de pouvoir recycler sans problème à l'atelier de production d'acrylate d'alkyle, la fraction acrylate d'alkyle/alcool générée sous forme d'azéotrope lors de la synthèse de l'ADAME, le recyclage de cette fraction étant une nécessité économique.

Un autre objectif de la présente invention est d'éviter que l'acétaldéhyde présent dans la fraction azéotropique provenant de la synthèse de l'ADAME ne génère de dialcoxyéthane dans l'atelier de production d'acrylate d'alkyle, qui s'ajouterait au dialcoxyéthane déjà présent dans la fraction azéotropique.

La présente invention vise un procédé de purification de la fraction azéotropique générée lors de la synthèse de l'ADAME particulièrement adapté au recyclage de cette fraction dans un procédé de synthèse d'acrylate d'alkyle, et ne nécessitant qu'un nombre modéré d'étapes.

La présente invention a pour objet un procédé de purification de la fraction azéotropique générée lors de la synthèse de l'acrylate de N,N-diméthyl aminoéthyle de formule (I) :

H₂C=CHCOOCH₂CH₂N(CH₃)₂ (I)

par transestérification d'un acrylate d'alkyle de formule (II) CH₂=CH-COOR₁ avec R₁ représentant le radical méthyl ou éthyl, par le N,N-diméthyl aminoéthanol, ladite fraction azéotropique comprenant de l'acrylate d'alkyle (II), l'alcool léger R₁OH formé lors de la réaction, et des sous-produits tels que de l'acétaldéhyde et du dialcoxyéthane de formule (III) CH₃-CH(OR₁)₂ étant distillée à l'aide d'une première colonne à distiller surmontant le réacteur de transestérification, caractérisé en ce que l'on effectue une distillation de ladite fraction à l'aide d'une seconde colonne à distiller visant à séparer les sous-produits acétaldéhyde et dialcoxyéthane, la fraction de tête minoritaire riche en acétaldéhyde, contenant de l'acrylate d'alkyle (II), de l'alcool léger R₁OH, la majeure partie de l'acétaldéhyde et des traces de dialcoxyéthane, étant éliminée, la fraction de pied majoritaire appauvrie en acétaldéhyde, contenant de l'acrylate d'alkyle (II), de l'alcool léger R₁OH, la majeure partie du dialcoxyéthane (II) et des traces d'acétaldéhyde, étant recyclée dans un procédé de synthèse d'acrylate d'alkyle (II) par réaction d'acide acrylique et d'alcool léger R₁OH.

Selon une alternative du procédé selon l'invention, la distillation de la fraction azéotropique visant à séparer les sous-produits acétaldéhyde et dialcoxyéthane est effectuée directement dans la première colonne à distiller surmontant le réacteur de transestérification, la fraction minoritaire riche en acétaldéhyde, contenant de l'acrylate d'alkyle (II), de l'alcool léger R₁OH, la majeure partie de l'acétaldéhyde et des traces de dialcoxyéthane, étant éliminée en tête, la fraction majoritaire appauvrie en acétaldéhyde, contenant de l'acrylate d'alkyle (II), de l'alcool léger R₁OH, la majeure partie du dialcoxyéthane (II) et des traces d'acétaldéhyde, étant extraite via un soutirage latéral de ladite première colonne.

Lors de la synthèse de l'ADAME par transestérification de l'acrylate de méthyle (ou de l'acrylate d'éthyle), on distille, lors de la phase réactionnelle, à l'aide d'une colonne à distiller surmontant le réacteur de transestérification une fraction azéotropique comprenant de l'acrylate de méthyle (ou de l'acrylate d'éthyle), du méthanol (ou de l'éthanol) et pouvant contenir en général de 100 ppm à 10000 ppm d'acétaldéhyde et de 100 ppm à 1000 ppm de dialcoxyéthane (diméthoxyéthane ou diéthoxyéthane suivant le cas).

Cette fraction contient en général, suivant l'efficacité de la colonne utilisée et les conditions de distillation, de 35% à 54% de méthanol ou de 60% à 72% d'éthanol.

Selon la première variante de l'invention, le procédé vise à n'éliminer que l'acétaldéhyde présent dans la fraction azéotropique, lequel est le contributeur très largement majoritaire à la formation ultérieure de dialcoxyéthane, lors du recyclage de la fraction azéotropique à l'atelier de production d'acrylate de méthyle ou d'acrylate d'éthyle.

Dans ce cas, les conditions de distillation sont réglées pour éliminer en tête de colonne tout l'acétaldéhyde, ou au moins 90%, tout en réduisant au maximum les pertes en acrylate léger et en alcool léger dans ce flux de tête de colonne riche en acétaldéhyde.

Il est possible d'utiliser une colonne à garnissage simple type anneaux Pall, ou structuré type Multiknit ou tout autre type de colonne. L'efficacité de la colonne doit être supérieure à 10 et avantageusement la colonne à distiller comporte de 10 à 20 plateaux théoriques, de préférence de 12 à 18 plateaux théoriques. La colonne à distiller est surmontée d'un condenseur alimenté par un fluide caloporteur chauffé à une température allant de 20°C à 50°C, de préférence de 25°C à 35°C. La colonne peut fonctionner de la pression atmosphérique à 2 bars, de préférence à pression atmosphérique.

Généralement, on introduit, dans l'azéotrope à distiller, au moins un inhibiteur de polymérisation, choisi par exemple parmi les inhibiteurs phénoliques (hydroquinone, éther méthylique d'hydroquinone, diterbutyl para crésol ...), la phénothiazine, les composé nitroxyls TEMPO type 4-OH ou 4-oxo TEMPO, etc. à raison de 100 à 5000 ppm.

Un bullage d'air ou d'air appauvri (7% O₂ en volume) est avantageusement introduit dans la colonne afin de renforcer l'action des inhibiteurs de polymérisation.

La fraction azéotropique épuisée en acétaldéhyde est récupérée en pied de la seconde colonne de distillation, ou peut être récupérée via un soutirage latéral directement sur la première colonne de distillation de l'azéotrope acrylate d'alkyle/alcool de l'atelier de production d'ADAME. Cette fraction ne pouvant plus générer de dialcoxyéthane peut être avantageusement recyclée.

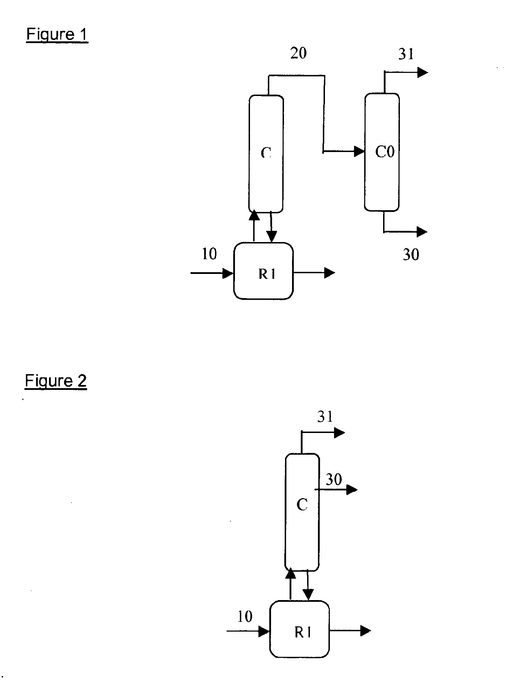

Cette variante de l'invention peut être illustrée selon les 2 configurations des figures 1 et 2 annexées..

Selon la configuration de la figure 1, le réacteur de transestérification R1, alimenté par les réactifs 10 (acrylate d'alkyle et DMAE), est surmonté d'une première colonne à distiller C permettant d'éliminer en continu en tête la fraction azéotropique 20 acrylate d'alkyle/alcool léger générée au cours de la réaction de transestérification. Selon l'invention, cette fraction 20 est distillée dans une seconde à colonne à distiller C0 d'où sont extraites, en tête une fraction 31 riche en acétaldéhyde, et en pied une fraction azéotropique purifiée 30. La colonne C0 comporte généralement de 10 à 20 plateaux théoriques (condenseur et bouilleur non inclus), l'alimentation se faisant généralement entre les plateaux 3 et 10, compté à partir du haut de la colonne (condenseur non compté).

Selon la configuration de la figure 2, la distillation de l'acétaldéhyde s'effectue directement dans la première colonne C située au-dessus du réacteur de transestérification R1. Il n'est pas nécessaire d'ajouter la seconde colonne à distiller, en adaptant les conditions de distillation de la colonne C, notamment un nombre de plateaux théoriques allant de 12 à 18, et en ajoutant une ligne de soutirage latéral dans la moitié supérieure de la colonne, par exemple au niveau 6 ou 7 dans le cas d'une colonne comportant 15 plateaux. La fraction 31 riche en acétaldéhyde sort en tête de la colonne C, tandis que le mélange azéotropique acrylate d'alkyle/alcool léger purifié 30 est extrait via ce soutirage latéral de cette colonne C.

Les conditons de fonctionnement des colonnes à distiller C et C0 seront adaptées par l'homme du métier pour éliminer de l'ordre de 90% de l'acétaldéhyde présent la fraction azéotropique et limiter les pertes en acrylate léger et alcool léger.

La fraction azéotropique purifiée 30 est alors avantageusement recyclée vers l'atelier de fabrication de l'acrylate d'alkyle.

Selon une seconde variante de l'invention, le procédé vise à rétrograder le dialcoxyéthane en acétaldéhyde, puis à éliminer celui-ci par distillation. Il en résulte ainsi une fraction azéotropique appauvrie à la fois en acétaldéhyde et en dialcoxyéthane.

La rétrogradation de l'équilibre d'acétalisation dans le sens de la formation de l'acétaldéhyde se fait en présence d'eau et d'un catalyseur acide, en distillant l'acétaldéhyde au fur et à mesure de sa formation.

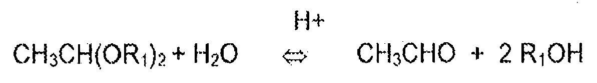

Cette réaction est connue, mais sa mise en oeuvre dans le cadre de la purification de la fraction azéotropique générée par la synthèse de l'ADAME n'a jamais été décrite dans l'art antérieur.

Selon cette seconde variante, on introduit dans la fraction azéotropique au moins un inhibiteur de polymérisation, choisi par exemple parmi les inhibiteurs phénoliques (hydroquinone, éther méthylique d'hydroquinone, diterbutyl para crésol ...), la phénothiazine, les composé nitroxyls TEMPO type 4-OH ou 4-oxo TEMPO, etc. à raison de 100 à 5000 ppm. Puis, on ajoute dans ce mélange de 3% à 20% massique d'eau ; de préférence, la quantité d'eau ajoutée est comprise entre 5 et 10% massique par rapport au mélange final

On ajoute également un catalyseur acide parmi lesquels on peut citer, les acides minéraux du type acide sulfurique, acide chlorhydrique, acide phosphorique, les acides sulfoniques tels que l'acide méthane sulfonique ou l'acide para toluène sulfonique, les résines cationiques sulfonées, ou les zéolithes fortement acides.

Les résines cationiques fortes sous forme gel ou macroporeuses sont préférées comme catalyseur acide, leur mise en oeuvre étant plus simple.

Le catalyseur peut être utilisé à raison de 5% à 20% massique par rapport à la quantité de fraction azéotrope à traiter. De préférence, on utilise de 10 à 15 % massique d'une résine cationique forte.

La quantité de catalyseur mise en oeuvre peut être inférieure à 5% au détriment du taux de réduction du dialcoxyéthane, ou supérieure à 20% sans que cela n'apporte un avantage notable.

Parmi les résines cationiques utilisables, on peut par exemple citer les résines Amberlyst 15, Lewatit K2620 ou K1461, Diaion PK228.

La mise en contact du catalyseur avec la fraction azéotropique à traiter peut se faire dans un réacteur agité mécaniquement et chauffé à l'aide d'une double-enveloppe dans laquelle circule un fluide thermostaté, sur un lit multiétagé de résine recirculé sur un rebouilleur externe, sur une cartouche de résine alimentée dans le sens ascendant ou descendant et placée sur une boucle de recirculation d'un réacteur, ou dans une colonne réactive.

Le procédé peut être effectué en discontinu (batch) ou en continu.

L'élimination en continu de l'acétaldéhyde est réalisée via une colonne à distiller, par exemple une colonne à plateaux, une colonne à garnissage vrac ou structuré.

Un bullage d'air ou d'air appauvri (8% O₂ en volume) est avantageusement introduit dans le mélange pendant toute l'opération afin de renforcer l'action des inhibiteurs de polymérisation.

La colonne à distiller est surmontée d'un condenseur alimenté par un fluide caloporteur chauffé à une température allant de 20°C et 50°C.

Les vapeurs non condensées, riches en acétaldéhyde, contiennent aussi de l'acrylate léger et de l'alcool. Elles sont ensuite condensées à l'aide d'une piège froid. Le taux de récupération de l'acétaldéhyde dans cette fraction de tête est généralement supérieur à 95%. Les pertes en acrylate léger (acrylate de méthyle ou acrylate d'éthyle et en alcool (méthanol ou éthanol) dans cette fraction sont fonction des conditions de distillation (efficacité de la colonne, conditions opératoires), elles sont généralement faibles en mettant en oeuvre la distilliation dans les conditions décrites précédemment.

Cette fraction riche en acétaldéhyde est éliminée et doit être minimisée car elle peut s'accompagner d'une perte en ester léger et alcool léger.

Le mélange acrylate léger / alcool léger débarrassé de tout ou partie de l'acétaldéhyde et du dialcoxyéthane peut ensuite être recyclé à l'atelier de production de l'acrylate léger sans risque de pollution de celui-ci.

Selon une troisième variante de l'invention, le procédé vise d'abord à extraire par de l'eau l'alcool léger et l'acétaldéhyde présents dans la fraction azéotropique puis à effectuer la distillation sur la fraction aqueuse provenant du lavage à l'eau. Ce lavage à l'eau est réalisé sur une colonne d'extraction à l'eau C1 alimentée à contre-courant.

La colonne d'extraction C1 reçoit la fraction azéotropique en pied et l'eau en tête. L'acrylate léger débarrassé de l'alcool léger et de l'acétaldéhyde est récupéré en tête. Différents types de colonnes d'extraction peuvent être utilisées. On peut à titre d'exemple utiliser une colonne compartimentée comprenant un arbre tournant avec un disque rotatif au milieu de chaque compartiment permettant une excellente mise en contact entre les phases.

La colonne d'extraction C1 peut être indépendante de l'atelier acrylates légers ou il peut s'agir d'une colonne type C1 déjà intégrée dans l'installation de productions d'acrylates légers.

On obtient en tête de la colonne d'extraction C1 une fraction contenant essentiellement l'acrylate d'alkyle (II) et plus ou moins de dialcoxyéthane, qui peut être recyclée avantageusement à la réaction de transestérification, et en pied de la colonne d'extraction C1 une fraction aqueuse contenant la majeure partie de l'eau ayant servi au lavage, l'alcool léger et l'acétaldéhyde, qui, après distillation, conduit d'une part en pied de colonne de distillation à une fraction aqueuse majoritaire appauvrie en acétaldéhyde et riche en alcool léger, ladite fraction pouvant alors être recyclée directement à l'étape de réaction entre l'acide acrylique et l'alcool léger dans un procédé de synthèse d'acrylate d'alkyle, d'autre part à une fraction de tête de colonne de distillation contenant la majeure partie de l'acétaldéhyde qui est ensuite éliminée.

Avantageusement, le dialcoxyéthane présent dans la fraction azéotropique pourra être transformé en acétaldéhyde, en présence d'eau et d'un catalyseur acide, comme décrit précédemment, avant l'extraction de l'alcool léger par de l'eau, permettant ainsi de conduire au final à une fraction exempte de dialcoxyéthane

Avantageusement, la distillation de la fraction aqueuse contenant l'alcool léger et l'acétaldéhyde obtenue en pied de la colonne d'extraction C1 est réalisée à l'aide de deux colonnes successives, la première colonne C2 servant à séparer l'eau qui se retrouve en pied de colonne C2 et qui est soit éliminée, soit recyclée au niveau de la colonne d'extraction C1, la fraction de tête de colonne C2 comprenant l'alcool et l'acétaldéhyde étant ensuite envoyée sur une seconde colonne à distiller C3, permettant de séparer une fraction de pied composée principalement d'alcool exempte d'acétaldéhyde qui peut être recyclée pour l'étape de réaction avec l'acide acrylique et une fraction de tête riche en acétaldéhyde qui sera détruite. Un mode de réalisation particulier de cette variante du procédé consiste à recycler la fraction de pied de colonne C3, composée principalement d'alcool et exempte d'acétaldéhyde, en partie pour l'étape de réaction avec l'acide acrylique et en partie en tête de colonne C2.

Un autre mode de réalisation particulier de cette variante du procédé consiste à renvoyer la majeure partie de la fraction de tête de colonne C3, qui contient l'acétaldéhyde, en reflux sur la colonne C3, le reste du flux étant purgé afin de déconcentrer l'installation en acétaldéhyde.

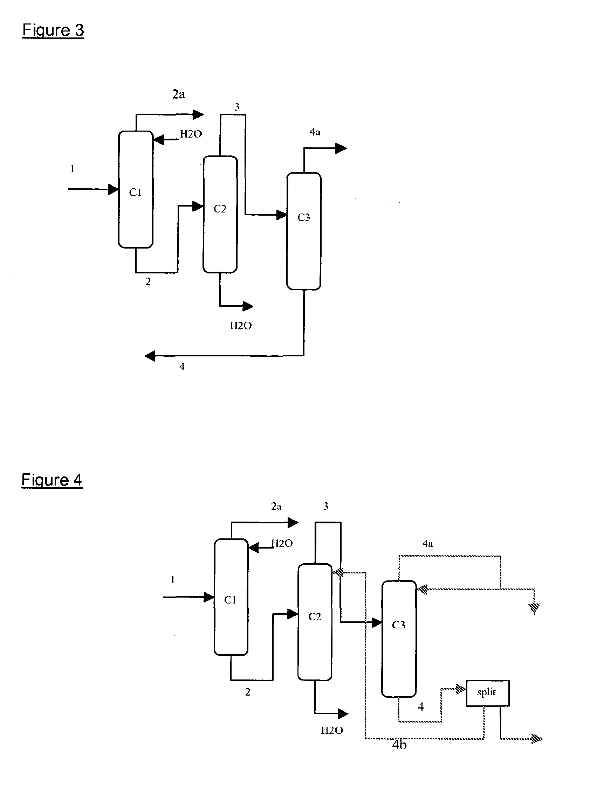

Cette variante de l'invention peut être illustrée selon les deux configurations des figures 3 et 4 annexées.

Selon la configuration de la figure 3, la fraction azéotropique 1 contenant l'acrylate léger, l'alcool, l'acétaldéhyde et plus ou moins de dialcoxyéthane est lavée par de l'eau dans une colonne C1 qui peut être intégrée ou non à l'atelier acrylates légers, d'où l'on extrait en tête un flux 2a comprenant essentiellement l'acrylate léger. Le flux 2, appauvri en acrylate léger et qui contient la totalité de l'acétaldéhyde, est envoyé sur une première colonne de distillation C2 d'où l'on sépare l'eau en pied et une fraction 3 constituée essentiellement d'alcool et d'acétaldéhyde. Cette fraction 3 est envoyée sur une colonne de distillation C3 qui permet d'éliminer tout l'acétaldéhyde contenu dans le flux 3. L'effluent 4 de pied de la colonne C3, débarrassé de l'acétaldéhyde est renvoyé vers la zone réactionnelle pour produire l'acrylate d'alkyle. La formation de dialcoxyéthane au niveau de la zone réactionnelle est de ce fait réduite, ce qui permet de limiter la teneur en dialcoxyéthane dans l'acrylate d'alkyle produit. Le flux 4a riche en acétaldéhyde est éliminé entraînant une perte inévitable en ester léger et alcool léger.

Selon la configuration de la figure 4, le flux 4a de tête de la colonne C3 est réparti de telle manière que la majorité du flux soit renvoyé en reflux sur la colonne C3 ; le reste du flux étant purgé afin de déconcentrer l'installation en acétaldéhyde. L'effluent 4 en pied de la colonne C3, appauvri en acétaldéhyde est renvoyé pour partie en tête de colonne C2 (flux 4b) et pour partie à la zone réactionnelle. En opérant selon la configuration de la figure 4, on augmente fortement la concentration en acétaldéhyde dans le coulage de la colonne C3 et on réduit de ce fait les pertes en réactifs (alcool léger / ester léger) du fait de la destruction imposée de cette fraction.

Avantageusement, la troisième variante du procédé de l'invention est réalisée directement sur l'unité de production d'acrylate d'alkyle léger, au niveau de l'étape de purification de l'acrylate brut issu du mélange réactionnel acide acrylique/alcool léger. Cela a pour avantage de ne pas traiter séparément la fraction azéotropique, et de ne pas nécessiter d'appareillages spécifiques pour ce traitement. La fraction azéotropique provenant de la production de l'ADAME est recyclée directement dans la colonne d'extraction à l'eau C1 de l'atelier de fabrication d'acrylates légers ; l'élimination de l'acétaldéhyde apporté par cette fraction, le recyclage de l'alcool présent dans cette fraction et la récupération de l'acrylate d'alkyle présent également dans cette fraction peuvent être réalisés en même temps que la purification de l'acrylate brut issu du mésange réactionnel. Pour cela, la fraction azéotropique est introduite au niveau d'une colonne d'extraction liquide/liquide alimentée par de l'eau, tout comme le mélange brut réactionnel débarrassé au préalable d'une partie de l'eau de la réaction, des composés lourds et de l'acide acrylique résiduel.

L'invention a aussi pour objet un procédé de synthèse de l'acrylate de N,N-diméthyl aminoéthyle de formule (I) :

H₂C=CHCOOCH₂CH₂N(CH₃)₂ (I)

par transestérification d'un acrylate d'alkyle de formule (II) CH₂=CH-COOR₁ avec R₁ représentant le radical méthyl ou éthyl, par le N,N-diméthyl aminoéthanol, dans lequel il est mis en oeuvre une étape de purification de la fraction azéotropique générée au cours de la réaction, selon les différentes variantes définies précédemment, permettant le recyclage de ladite fraction sur l'unité de production de l'acrylate d'alkyle.

Les exemples ci-après illustrent la présente invention sans toutefois en limiter la portée.

### EXEMPLES

Les pourcentages sont exprimés en pourcentages massiques.

Les abréviations suivantes sont utilisées :
- AE : acrylate d'éthyle
- AM : acrylate de méthyle
- EMHQ : ester méthylique d'hydroquinone

### Exemples 1 à 4

Dans un réacteur à double-enveloppe alimentée par de l'huile à 80°C et surmonté d'une colonne à distiller à garnissage Multiknit (efficacité :15 plateaux théoriques), avec condenseur en tête de colonne alimenté par de l'huile à 50°C et piège à carboglace, on charge :
- 500g ou 550g de fraction à traiter (contenant 40% d'AE ; 59,5% d'éthanol ; acétaldéhyde ; diéthoxyéthane)
- 0 ou 50g de résine Lewatit K1461 ou Amberlyst 15
- 0 ou 50g d'eau
- 0,1% EMHQ
la masse totale fraction à traiter + eau étant égale à 550g.

On maintient un bullage d'air appauvri à 8% O₂ (vol) pendant toute la durée de l'essai (3 heures).

On chauffe à ébullition à 63°C en éliminant l'acétaldéhyde au fur et à mesure de sa formation par distillation.

Les teneurs en acétaldéhyde et en diéthoxyéthane sont déterminées par chromatographie en phase gazeuse.

| | Type de résine | Eau g | Fraction à traiter, g | Masse après traitement, g | Teneur en acétaldéhyde, ppm | | Teneur en diéthoxyéthane, ppm | |
|---|---|---|---|---|---|---|---|---|
| | | | | | initiale | finale | Initiale | finale |
| Ex 1 | Néant | 50 | 500 | 450 | 333 | 20 | 319 | 303 |
| Ex 2 | K1461 | 50 | 500 | 385 | 364 | 23 | 297 | 18 |
| Ex 3 | A15 | 50 | 500 | 400 | 303 | 12 | 319 | 10 |
| Ex 4 | K1461 | 0 | 550 | 430 | 383 | 56 | 356 | 264 |

On constate qu'en l'absence de catalyseur acide ou en l'absence d'eau, le diéthoxyéthane n'est pas transformé en acétaldéhyde et est donc difficilement éliminé par distillation. Le taux d'élimination de l'acétaldéhyde par distillation est dans presque tous les cas supérieur à 90%.

### Exemple 5

Le mélange précédent de l'exemple 2 (500g de fraction à traiter et 50g d'eau) est traité dans un réacteur agité chauffé par double-enveloppe pourvu d'une recirculation sur une cartouche contenant 200 ml de résine LEWATIT K1461. Le réacteur est surmonté d'une colonne à garnissage Multiknit d'efficacité égale à 20 plateaux théoriques muni d'un condenseur alimenté par de l'eau à 30°C.

Le mélange est alimenté dans le réacteur à un débit de 1500 g/h.
L'acétaldéhyde est distillé en tête de colonne à environ 30 g/h. La composition de la fraction de tête est la suivante :
AE : 28%
Ethanol : 66%
Acétaldéhyde : 0,06%

Le mélange final contient :
AE : 40%
Ethanol : 59,5%
Diéthoxyéthane : 180 ppm

Le taux d'élimination du diéthoxyéthane potentiel dans le mélange avant traitement est supérieur à 90%

### Exemple 6 (en référence à la figure 1)

Le mélange azéotropique suivant contenant :
AE : 40%
Ethanol : 60%
Acétaldéhyde : 0,023%
Diéthoxyéthane : 0,05%
est distillé sur une colonne C0 garnie d'anneaux Pall 2,54 cm (1") ayant les caractéristiques suivantes: diamètre 32 cm ; hauteur 5 m ; efficacité 10 plateaux théoriques.

La température au niveau du condenseur est d'environ 30°C. La colonne est munie également d'un piège à -10°C.

Le mélange est alimenté au plateau 4 de la colonne (compté à partir du haut) à un débit de 1425 kg/h.

La fraction de tête contenant l'acétaldéhyde distille à 74°C à Pression Atmosphérique à 4 kg/h.

Sa composition massique est la suivante :
AE : 27,7%
Ethanol : 65%
Acétaldéhyde : 7,3%
Diéthoxyéthane : 0,02 %

On récupère en pied un mélange composé de :
AE : 40%
Ethanol : 60%
Acétaldéhyde : 3 ppm
Diéthoxyéthane : 0,05%

La fraction de tête qui correspond à 0,3 % du mélange initial, est éliminée, ce qui représente une perte en AE très faible et un taux de récupération de l'acétaldéhyde de 90%.

La fraction de pied, épuisée en acétaldéhyde, contributeur majoritaire à l'introduction de diéthoxyéthane dans l'atelier AE par le biais du recyclage, peut être recyclée à cet atelier.

### Exemple 7 (en référence à la figure 2)

Dans un réacteur R1 agité chauffé, surmonté d'une colonne à distiller C garnie de 2 lits de 3 et 5 m et comportant 15 plateaux théoriques, on introduit le mélange suivant (7745 kg/h) :
- AE : 29,7 %
- DMAE : 13,8 %
- ADAME: 37 %
- EtOH: 12,4 %
- Acétaldéhyde: 44 ppm
- Lourds : 5 %

La température du condenseur est d'environ 30°C et celle du piège d'évents de -8°C.

La pression en tête de colonne est de 86,66 kPa (650 mmHg).

La fraction riche en acétaldéhyde est distillée en tête de colonne tandis que l'azéotrope purifié est soutiré au plateau théorique 7 (compté à partir du condenseur). Ceci permet d'avoir une pureté en éthanol supérieure à 66% et d'éliminer plus de 90% de l'acétaldéhyde présent.

Le coulage de tête (1 kg/h) est constitué de :
- acétaldéhyde : 21,2 %
- AE : 23,5%
- Ethanol : 54,8%

Le soutirage latéral (1217 kg/h) est constitué de :
- AE : 33,9 %
- Ethanol : 66 %
- Acétaldéhyde : 0,0014 %

### Exemple 8 (en référence à la figure 3)

La colonne C3 (au plateau 7 à partir du bas) est alimentée à 800 kg/h par un flux 3 ayant la composition massique suivante :
AE : 17%
Ethanol : 74%
Eau : 8%
Acétaldéhyde : 2800 ppm

La colonne fonctionne dans les conditions suivantes :
P : 2 bars - T° tête : 65°C - T° pied : 79°C - efficacité de colonne : 11 plateaux théoriques - taux de reflux : 12/1.

On obtient :
- un flux de tête 4a : 17 kg/h, constitué de :
   AE : 47%
   Ethanol : 35%
   eau : 5%
   Acétaldéhyde : 13%
- un flux de pied 4 : 783 kg/h, constitué de
   AE : 16,1%
   Ethanol : 75,7%
   eau : 8,2%

### Exemple 9 (en référence à la figure 4)

Le flux qui alimente la colonne C2 à raison de 349,3 kg/h a la composition massique suivante :
AE : 5,8%
Ethanol : 21,9%
eau : 72,1%
Acétaldéhyde : 1500 ppm (0,52 kg/h)

L'alimentation de la colonne C3 se fait au plateau 6. L'efficacité de cette colonne est de 7 plateaux théoriques.

La purge en tête de la colonne C3 est de 1 kg/h. Elle est volontairement limitée afin de ne pas perdre trop d'AE et d'éthanol.

Le débit massique d'acétaldéhyde purgé en tête de colonne C3 est de 476 g/h. Le débit massique d'acétaldéhyde renvoyé à la réaction est de 44 g/h soit un taux de réduction de la quantité d'acétaldéhyde dans l'installation de 91,5%.

## Revendications

1. Procédé de purification de la fraction azéotropique générée lors de la synthèse de l'acrylate de N,N-diméthyl aminoéthyle de formule (I) :
H₂C=CHCOOCH₂CH₂N(CH₃)₂ (I)
par transestérification d'un acrylate d'alkyle de formule (II) CH₂=CH-COOR₁ avec R₁ représentant le radical méthyl ou éthyl, par le N,N-diméthyl aminoéthanol, ladite fraction azéotropique comprenant de l'acrylate d'alkyle (II), l'alcool léger R₁OH formé lors de la réaction, et des sous-produits tels que de l'acétaldéhyde et du dialcoxyéthane de formule (III) CH₃-CH(OR₁)₂ étant distillée à l'aide d'une première colonne à distiller surmontant le réacteur de transestérification, **caractérisé en ce que** l'on effectue une distillation de ladite fraction visant à séparer les sous-produits acétaldéhyde et dialcoxyéthane, et
(a) soit, l'étape de distillation est effectuée à l'aide d'une seconde colonne à distiller, la fraction de tête minoritaire riche en acétaldéhyde, contenant de l'acrylate d'alkyle (II), de l'alcool léger R₁OH, la majeure partie de l'acétaldéhyde et des traces de dialcoxyéthane étant éliminée, la fraction majoritaire appauvrie en acétaldéhyde, contenant de l'acrylate d'alkyle (II), de l'alcool léger R₁OH, la majeure partie du dialcoxyéthane (II) et des traces d'acétaldéhyde étant récupérée en pied de cette seconde colonne,
(b) soit, l'étape de distillation est effectuée directement dans la première colonne à distiller, la fraction de tête minoritaire riche en acétaldéhyde, contenant de l'acrylate d'alkyle (II), de l'alcool léger R₁OH, la majeure partie de l'acétaldéhyde et des traces de dialcoxyéthane étant éliminée, la fraction majoritaire appauvrie en acétaldéhyde, contenant de l'acrylate d'alkyle (II), de l'alcool léger R₁OH, la majeure partie du dialcoxyéthane (II) et des traces d'acétaldéhyde, étant extraite via un soutirage latéral de ladite première colonne,
la fraction appauvrie en acétaldéhyde étant recyclée dans un procédé de synthèse d'acrylate d'alkyle (II) par réaction d'acide acrylique et d'alcool léger R₁OH.

2. Procédé selon la revendication 1 **caractérisée en ce que** l'étape de distillation visant à séparer les sous-produits acétaldéhyde et dialcoxyéthane est réalisée directement dans la colonne de distillation de la fraction azéotropique surmontant le réacteur de transestérification (voie (b)).

3. Procédé selon la revendication 1 **caractérisée en ce que** l'étape de distillation visant à séparer les sous-produits acétaldéhyde et dialcoxyéthane est réalisée à l'aide d'une seconde colonne à distiller (voie (a)).

4. Procédé selon la revendication 3 **caractérisé en ce que** l'on soumet la fraction azéotropique à un traitement préalable avec de l'eau en présence d'un catalyseur acide, le dialcoxyéthane étant alors transformé en acétaldéhyde et alcool léger, et l'on distille l'acétaldéhyde au fur et à mesure de sa formation.

5. Procédé selon la revendication 3 ou 4 **caractérisé en ce que** l'on effectue la distillation sur la fraction aqueuse provenant du lavage à l'eau de la fraction azéotropique sur une colonne d'extraction C1, ladite fraction aqueuse contenant la majeure partie de l'eau ayant servi au lavage, l'alcool léger et l'acétaldéhyde étant obtenue en pied de la colonne d'extraction C1, la fraction obtenue en tête de la colonne d'extraction C1 qui contient essentiellement l'acrylate d'alkyle (II) et plus ou moins de dialcoxyéthane pouvant être recyclée directement à la réaction de transestérification, la fraction de pied de colonne de distillation majoritaire appauvrie en acétaldéhyde et riche en alcool léger étant alors recyclée directement à l'étape de réaction entre l'acide acrylique et l'alcool léger dans un procédé de synthèse d'acrylate d'alkyle, la fraction de tête de la colonne de distillation contenant la majeure partie de l'acétaldéhyde étant éliminée.

6. Procédé selon la revendication 5 **caractérisé en ce que** l'on effectue la distillation à l'aide de deux colonnes de distillation successives C2 et C3, la première colonne C2 servant à séparer l'eau qui se retrouve en pied de colonne et qui est soit éliminée, soit recyclée au niveau de la colonne d'extraction C1, la fraction de tête de colonne comprenant l'alcool et l'acétaldéhyde étant ensuite envoyée sur une seconde colonne à distiller C3, permettant de séparer en pied une fraction alcool exempte d'acétaldéhyde qui peut être recyclée pour l'étape de réaction avec l'acide acrylique, la fraction de tête qui contient l'acétaldéhyde étant éliminée.

7. Procédé selon la revendication 6 **caractérisé en ce que** la fraction de pied de colonne C3, composée principalement d'alcool et exempte d'acétaldéhyde, est recyclée en partie pour l'étape de réaction avec l'acide acrylique et en partie en tête de colonne C2.

8. Procédé selon la revendication 6 ou 7 **caractérisé en ce que** la majeure partie de la fraction de tête de colonne C3, qui contient l'acétaldéhyde, est renvoyée en reflux sur la colonne C3, le reste du flux étant purgé afin de déconcentrer l'installation en acétaldéhyde.

9. Procédé selon l'une quelconque des revendications 5 à 8 **caractérisé en ce que** la phase de lavage à l'eau de la fraction azéotropique est réalisée directement sur l'unité de production d'acrylate d'alkyle (II), au niveau de l'étape de purification de l'acrylate brut issu du mélange réactionnel acide acrylique/alcool, la fraction azéotropique étant introduite au niveau de la colonne d'extraction liquide/liquide alimentée par de l'eau, déjà intégrée dans l'installation de production de l'acrylate d'alkyle, tout comme le mélange brut réactionnel débarrassé au préalable d'une partie de l'eau de la réaction, des composés lourds et de l'acide acrylique résiduel.

10. Procédé de synthèse de l'acrylate de N,N-diméthyl aminoéthyle de formule (I) :
H₂C=CHCOOCH₂CH₂N(CH₃)₂ (I)
par transestérification d'un acrylate d'alkyle de formule (II) CH₂=CH-COOR₁ avec R₁ représentant le radical méthyl ou éthyl, par le N,N-diméthyl aminoéthanol, **caractérisé en ce qu'**il comprend une étape de purification de la fraction azéotropique générée au cours de la réaction, telle que définie selon le procédé de l'une des revendications précédentes, permettant le recyclage de ladite fraction sur l'unité de production de l'acrylate d'alkyle.

## Claims

1. A method for purifying the azeotropic fraction generated during the synthesis of N,N-dimethyl aminoethyl acrylate of formula (I):
H₂C=CHCOOCH₂CH₂N(CH₃)₂ (I)
by transesterification of an alkyl acrylate of formula (II) CH₂=CH-COOR₁ with R₁ representing the methyl or ethyl radical, by N,N-dimethyl aminoethanol, said azeotropic fraction comprising alkyl acrylate (II), the lower alcohol R₁OH formed during the reaction, and byproducts such as acetaldehyde and dialkoxyethane of formula (III) CH₃-CH(OR₁)₂ being distilled by means of a first distillation column mounted above the transesterification reactor, **characterized in that** said fraction is distilled with a view to separating the byproducts acetaldehyde and dialkoxyethane, and
(a) either, the distillation step is carried out by means of a second distillation column, the minority acetaldehyde-rich overhead product, containing alkyl acrylate (II), lower alcohol R₁OH, most of the acetaldehyde and traces of dialkoxyethane being removed, and the low-acetaldehyde main fraction, containing alkyl acrylate (II), lower alcohol R₁OH, most of the dialkoxyethane (II) and traces of acetaldehyde being recovered at the bottom of said second column,
(b) or, the distillation step is carried out directly in the first distillation column, the minority acetaldehyde-rich overhead product, containing alkyl acrylate (II), lower alcohol R₁OH, most of the acetaldehyde and traces of dialkoxyethane being removed, and the low-acetaldehyde main fraction, containing alkyl acrylate (II), lower alcohol R₁OH, most of the dialkoxyethane (II) and traces of acetaldehyde, being drawn off via a side stream of said first column,
the low-acetaldehyde fraction being recycled to a synthesis process for alkyl acrylate (II) by reaction of acrylic acid and lower alcohol R1OH.

2. The method as claimed in claim 1, **characterized in that** the distillation step for separating the byproducts acetaldehyde and dialkoxyethane is carried out directly in the column for distillation of the azeotropic fraction mounted abode the transesterification reactor (route (b)).

3. The method as claimed in claim 1, **characterized in that** the distillation step for separating the byproducts acetaldehyde and dialkoxyethane is carried out by means of a second distillation column (route (a)).

4. The method as claimed in claim 3, **characterized in that** the azeotropic fraction is submitted to a preliminary treatment with water in the presence of an acid catalyst, the dialkoxyethane then being transformed to acetaldehyde and lower alcohol, and the acetaldehyde is distilled as it forms.

5. The method as claimed in claim 3 or 4, **characterized in that** distillation of the aqueous fraction from washing the azeotropic fraction with water is carried out on an extraction column C1, said aqueous fraction containing most of the water that was used for washing, the lower alcohol and acetaldehyde being obtained at the bottom of extraction column C1, the fraction obtained at the top of extraction column C1 which contains essentially alkyl acrylate (II) and a variable amount of dialkoxyethane that can be recycled directly to the transesterification reaction, the main bottom product of the distillation column, depleted of acetaldehyde and rich in lower alcohol, then being recycled directly to the reaction steep between the acrylic acid and the lower alcohol in an alkyl acrylate synthesis process, the overhead product of the distillation column containing most of the acetaldehyde being removed.

6. The method as claimed in claim 5, **characterized in that** distillation is performed by means of two successive distillation columns C2 and C3, the first column C2 serving for separating the water occurring at the bottom of the column and which is either removed, or is recycled to extraction column C1, the overhead product of the column comprising alcohol and acetaldehyde then being sent to a second distillation column C3, separating, at the bottom, an acetaldehyde-free alcohol fraction, which can be recycled to the reaction step with acrylic acid, the overhead product which contains acetaldehyde being removed.

7. The method as claimed in claim 6, **characterized in that** the bottom product of column C3, composed mainly of alcohol and free from acetaldehyde, is recycled partly to the reaction steep with acrylic acid and partly to the top of column C2.

8. The method as claimed in claim 6 or 7, **characterized in that** most of the overhead product of column C3, which contains acetaldehyde, is returned in reflux to column C3, the rest of the stream being purged in order to lower the concentration of acetaldehyde in the plant.

9. The method as claimed in any one of claims 5 to 8, **characterized in that** the phase of washing the azeotropic fraction with water is carried out directly in the production unit for alkyl acrylate (II), at the level of the step for purification of the raw acrylate obtained from the acrylic acid/alcohol reaction mixture, the azeotropic fraction being introduced at the level of the liquid/liquid extraction column supplied with water, already integrated in the plant for production of alkyl acrylate, as well as the raw reaction mixture after prior removal of a proportion of the water from the reaction, heavy compounds and residual acrylic acid.

10. A method of synthesis of N,N-dimethyl aminoethyl acrylate of formula (I):
H₂C=CHCOOCH₂CH₂N(CH₃)₂ (I)
by transesterification of an alkyl acrylate of formula (II) CH₂=CH-COOR₁ with R₁ representing the methyl or ethyl radical, by N,N-dimethyl aminoethanol, **characterized in that** it comprises a step of purification of the azeotropic fraction generated during the reaction, as defined according to the method of one of the preceding claims, permitting recycling of said fraction to the alkyl acrylate production unit.

## Patentansprüche

1. Verfahren zur Reinigung der bei der Synthese von N,N-Dimethylaminoethylacrylat der Formel (I):
H₂C=CHCOOCH₂CH₂N(CH₃)₂ (I)
durch Umesterung eines Alkylacrylates der Formel (II) CH₂=CH-COOR₁, wobei R₁ für den Methyl- oder Ethylrest steht, mit N,N-Dimethylaminoethanol anfallenden azeotropen Fraktion, wobei die azeotrope Fraktion, die Alkylacrylat (II), den bei der Reaktion gebildeten niederen Alkohol R₁OH und Nebenprodukte wie Azetaldehyd und Dialkoxyethan der Formel (III) CH₃-CH(OR₁)₂ umfasst, mit Hilfe einer ersten Destillationskolonne, die auf den Umesterungsreaktor ausgesetzt ist, destilliert wird, **dadurch gekennzeichnet, dass** man eine Destillation der Fraktion zwecks Abtrennung der Nebenprodukte Acetaldehyd und Dialkoxyethan durchführt rund
(a) entweder den Destillationsschritt mit Hilfe einer zweiten Destillationskolonne durchführt, wobei man die arm Kopf anfallende acetaldehydreiche Nebenfraktion, die Alkylacrylate (II), niederen Alkohol R₁OH, den größten Teil des Acetaldehyds und Spuren von Dialkoxyethan enthält, ausschleust und die acetaldehydarme Hauptfraktion, die Alkylacrylat (II), niederen Alkohol R₁OH, den größten Teil des Dialkoxyethans (II) und Spuren von Acetaldehyd enthält, am Sumpf dieser zweiten Kolonne gewinnt,
(b) oder den Destillationsschritt direkt in der ersten Destillationskolonne durchführt, wobei man die am Kopf anfallende acetaldehydreiche Nebenfraktion, die Allylacrylat (II), niederen Alkohol R₁OH, den größten Teil des Acetaldehyds und Spuren von Dialkoxyethan enthält, ausschleust und die acetaldehydarme Hauptfraktion, die Allylacrylat (II), niederen Alkohol R₁OH, den größten Teil des Dialkoxyethans (II) und Spuren von Acetaldehyd enthält, über einen Seitenabzug der ersten Kolonne abzieht,
wobei die acetaldehydarme Fraktion zu einem Verfahren zur Synthese von Alkylacrylat (II) durch Umsetzung von Acrylsäure mit dem niederen Alkohol R₁OH rezykliert wird.

2. Verfahren nach Anspruch 1, **dadurch gekennzeichnet, dass** man den Schritt der Destillation zwecks Abtrennung der Nebenprodukte Acetaldehyd und Dialkoxyethan direkt in der auf den Umesterungsreaktor ausgesetzten Destillationskolonne für die azeotrope Fraktion durchführt (Weg (b)).

3. Verfahren nach Anspruch 1, **dadurch gekennzeichnet, dass** man den Schritt der Destillation zwecks Abtrennung der Nebenprodukte Acetaldehyd und Dialkoxyethan mit Hilfe einer zweiten Destillationskolonne durchführt (Weg (a)).

4. Verfahren nach Anspruch 3, **dadurch gekennzeichnet, dass** man die azeotrope Fraktion einer Vorbehandlung mit Wasser in Gegenwart eines Säurekatalysetors unterwirft, wobei das Dialkoxyethan dann in Acetaldehyd und niederen Alkhol umgewandelt wird, und das Acetaldehyd in dem Maße, wie es gebildet wird, abdestilliert.

5. Verfahren nach Anspruch 3 oder 4, **dadurch gekennzeichnet, dass** man die Destillation an der aus der Wäsche der azeotropen Fraktion mit Wasser stammenden wässrigen Fraktion an einer Extraktionskolonne C1 durchführt, wobei arm Sumpf der Extraktionskolonne C1 die wässrige Fraktion, die den größten Teil des für das Waschen verwendeten Wassers, niederen Alkohol und Azetaldehyd enthält, erhalten wird, wobei die arm Kopf der ExtraktionsKolonne C1 erhaltene Fraktion, die im Wesentlichen Alkylacrylat (II) und mehr oder weniger Dialkoxyethan enthält, direkt zur Umesterungsreaktion rezykliert werden kann, wobei die am Sumpf der Destillationskolonne anfallende acetaldehydarme und an niederem Alkohol reiche Hauptfraktion dann direkt in den Schritt der Reaktion zwischen Acrylsäure und dem niederen Alkohol in einem Verfahren zur Synthese von Allylacrylat rezykliert wird, wobei die Kopffraktion der Destillationskolonne, die den größten Teil des Acetaldehyds enthält, ausgeschleust wird.

6. Verfahren nach Anspruch 5, **dadurch gekennzeichnet, dass** man die Destillation mit Hilfe von zwei aufeinanderfolgenden Destillationskolonnen C2 und C3 durchführt, wobei die Kolonne C2 zur Abtrennung des am Sumpf der Kolonne anzutreffenden Wassers, das entweder ausgeschleust oder zur Extraktionskolonne C1 rezykliert wird, dient, wobei die Kopffraktion der Kolonne, die Alkohol und Acetaldehyd umfasst, dann einer zweiten Destillationskolonne C3 zugeführt wird, die die Abtrennung einer acetaldehydfreien Alkoholfraktion am Sumpf ermöglicht, welche für den Schritt der Umsetzung mit Acrylsäure rezykliert werden kann, wobei die Kopffraktion, die Azetaldehyd enthält, ausgeschleust wird.

7. Verfahren nach Anspruch 6, **dadurch gekennzeichnet, dass** man die Kopffraktion der Kolonne C3, die hauptsächlich aus Alkohol besteht und frei von Acetaldehyd ist, teilweise für den Schritt der Umsetzung mit Acrylsäure und teilweise zum Kopf von Kolonne C2 rezykliert.

8. Verfahren nach Anspruch 6 oder 7, **dadurch gekennzeichnet, dass** man den größten Teil der Kopffraktion von Kolonne C3, die Acetaldehyd enthält, im Rücklauf zur Kolonne C3 zurückführt, wobei der Rest des Stroms zur Herabsetzung der Acetaldehydkonzentration in der Anlage abgelassen wird.

9. Verfahren nach einem der Absprüche 5 bis 8, **dadurch gekennzeichnet, dass** man die Phase des Waschens der azeotropen Fraktion mit Wasser direkt in der Einheit zur Herstellung von Alkylacrylat (II) auf dem Niveau des Schritts der Reinigung des aus der Acrylsäure/Alkohol-Reaktionsmischung erhaltenen Rohacrylats durchführt, wobei die azeotrope Fraktion auf dem Niveau der wassergespeisten Flüssig/Flüssig-Extraktionskolonne, die bereits in die Anlage zur Herstellung von Allylacrylat integriert ist, eingetragen wird, ebenso wie die zuvor von einem Teil des Reaktionswassers, der schweren Verbindungen und der restlichen Acrylsäure befreite rohe Reaktionsmischung.

10. Verfahren zur Synthese von N,N-Dimethylaminoethylacrylat der Formel (I) :
H₂C=CHCOOCH₂CH₂N(CH₃)₂ (I)
durch Umesterung eines Alkylacrylates der Formel (II) CH₂=CH-COOR₁, wobei R₁ für den Methyl- oder Ethylrest steht, mit N,N-Dimethylaminoethanol, **dadurch gekennzeichnet, dass** es einen Schritt der Reinigung der im Lauf der Reaktion anfallenden azeotropen Fraktion, wie er gemäß dem Verfahren nach einem der vorhergehenden Ansprüche definiert ist, umfasst, was die Rezyklierung der Fraktion zu einer Einheit zur Herstellung von Alkylacrylat ermöglicht.
